# EUROPÄISCHE PATENTSCHRIFT

(11) **EP 2 682 080 B1**
(45) Veröffentlichungstag und Bekanntmachung des Hinweises auf die Patenterteilung: **03.02.2016**
(21) Anmeldenummer: 13174506.9
(22) Anmeldetag: 01.07.2013
(51) Int. Cl.: A61F 9/008, A61B 18/26, A61N 5/067

(54) **Applikator und Vorrichtung zur Zellbehandlung**
Applicator and device for cell treatment
Applicateur et dispositif de traitement de cellules

(30) Priorität: 05.07.2012 DE 102012106017
(43) Veröffentlichungstag der Anmeldung: 08.01.2014
(73) Patentinhaber: A.R.C. Laser GmbH, 90411 Nürnberg (DE)
(72) Erfinder: Thyzel, Reinhardt, 90542 Eckental (DE)
(74) Vertreter: Schröer, Gernot H.

(56) Entgegenhaltungen:
- WO-A1-99/04737
- US-A1- 2004 158 236

## Beschreibung

Die Erfindung betrifft einen Applikator und eine Vorrichtung jeweils zur Zellbehandlung, insbesondere Epithelzellenentfernung oder -inaktivierung.

Aus der US 2002/0058890 A1 ist eine Vorrichtung zur Lithotripsie bekannt, welche im Innenraum eines rohrförmigen Gehäuses eine optische Faser und einen Absorber umfasst Die optische Faser ist an einem ersten Ende mit einem Laser verbunden ist, und an ihrem anderen zweiten Ende mit einer dem Innenraum des Gehäuses zugewandten Innenseite des Absorbers optisch gekoppelt ist. Bei Auftreffen von Laserstrahlung des Lasers auf den Absorber an dessen dem Innenraum des Gehäuses Innenseite erzeugt der Absorber eine Druckwelle, welche an dessen Außenseite dann durch eine das zweite Ende und den Absorber umgreifende Endkappe transmittiert und abgestrahlt wird. Durch unterschiedliche Geometrien der Endkappe, etwa nach Art von Linsen oder Kavitäten, können unterschiedliche Formen und/oder Ausbreitungsrichtungen der Druckwelle erreicht werden. Das rohrförmige Gehäuse weist keine Austrittsöffnung für die Druckwelle auf, der Absorber schließt das rohrförmige Gehäuse an dessen freien Ende ab. Ein an der Innenseite des Absorbers durch das Laserstrahlung eventuell entstehendes Plasma verbleibt dadurch im Innenraum des Gehäuses.

Aus der WO 2004/071319 A1 ist eine chirurgische Hohlnadel zur Augenkataraktbehandlung mit einer darin geführten optischen Faser bekannt, wobei die optische Faser ist an ihrem außerhalb der Hohlnadel gelegenen ersten Ende mit einem Laser gekoppelt ist und am zweiten Ende der optischen Faser im Bereich und beabstandet von der Spitze der Hohlnadel gelegen ist. Im Bereich der Spitze der Hohlnadel weist diese ein Target auf, das aus einem Bereich der Wandung der Hohlnadel an der Spitze gebildet ist und an dem bei Bestrahlung mit Laserstrahlung des Lasers aus der Faser ein Plasma erzeugt wird, wodurch wiederum eine Druckwelle entsteht. An der Spitze der Hohlnadel ist eine Öffnung vorgesehen, durch die die Druckwelle nach außen treten kann. Die Mantel- oder Seitenwand der Hohlnadel ist monolithisch mit der Spitze aus Titan (Ti) gebildet und als durchgehende, glatte Wandung ohne Öffnung ausgebildet.

Aus US 5,324,282 A ist ein chirurgisches Instrument in Form einer Hohlnadel zum Entfernen der Augenlinse durch Photolyse bekannt, das eine rohrförmige Außenwand mit einer longitudinalen Achse und einem freien Ende sowie eine Laserfaser und einen Absaugkanal, die jeweils longitudinal und im Innenraum der Nadel bis zu deren freien Ende verlaufen, aufweist. Am freien Ende der Nadel ist ein Target aus Titan (Ti) angeordnet in einem Abstand zum freien Ende der Laserfaser, wobei das Laserlicht aus der Laserfaser auf das Target trifft. An dem freien Ende der Nadel ist ferner eine schräg und oberhalb des Targets unmittelbar neben diesem angeordnete Gewebeaufnahmeöffnung vorgesehen, in die der Absaugkanal mündet. Über eine Absaugpumpe wird das zu zerstörende Gewebe an die Gewebeaufnahmeöffnung angesaugt. Wenn nun das Gewebe an der Gewebeaufnahmeöffnung angesaugt ist, wird das Target mit Laserpulsen aus der Laserfaser beschossen, wobei die Laserpulse eine ausreichende Energie haben, um einen optischen Zusammenbruch (optical breakdown) im Targetmaterial zu erzeugen und damit eine Stoßwelle oder Schockwelle (shockwave) zu erzeugen, die an der Gewebeaufnahmeöffnung auf das dort befindliche Gewebe trifft und dieses in kleine Stücke reißt, die dann durch den Absaugkanal abgesaugt werden. Ferner kann in der Nadel zusätzlich ein longitudinal verlaufender Spülkanal zum Leiten von Spülflüssigkeit durch eine seitlich angeordnete Austrittsöffnung vorgesehen sein.

In US 5,906,611 A ist eine Weiterbildung des aus US 5,324,282 A bekannten Instruments bekannt, bei der das Target stufenförmig ausgebildet ist und bei der die Stufen mit zwei Stufenflächen, von denen eine senkrecht zur Nadelachse und die andere parallel zur Nadelachse gerichtet sind, ausgebildet sind und die Abfolge der Stufen von einer Außenseite an der Außenwand der Nadel zur Gewebeaufnahmeöffnung hin ansteigt. Dadurch wird in jeder Stufenzone des Targets bei der Verdampfung des Targetmaterials die dadurch erzeugte Stoßwelle nicht in Richtung zur Gewebeaufnahmeöffnung hin von einem anderen Teil des Targets blockiert. Mit einem Neodym-YAG-Laser können Pulse erzeugt werden mit Pulswiederholungsraten zwischen 2 und 50 Pulsen pro Sekunde und Pulsenergien zwischen 2 und 15 mJ. Die Pulsdauer kann zwischen 8 und 12 ns eingestellt sein. Vorzugsweise ist die Pulswiederholungsrate zwischen 2 und 6 Pulsen pro Sekunde und die Pulsenergie zwischen 6 und 10 mJ eingestellt. Für eine Kataraktoperation werden zwischen 200 und 800 Pulse verwendet.

Ein Problem bei der operativen Explantation oder Phakolyse der natürlichen Augenlinse und der anschließenden Implantation einer künstlichen intraokularen Linse ist das nachträgliche Wachstum und Wuchern der Epithelzellen an der Innenseite des Linsenkapselsacks (PCO für *posterior capsule opacification* oder sekundärer Katarakt oder Nachstar).

Die WO 2005/107665 A1 offenbart eine Vorrichtung zum Entfernen von Epithelzellen von der Innenseite eines Linsenkapselsackes eines menschlichen oder tierischen Auges mit Mitteln zum Erzeugen von Druckpulsen in einem flüssigen Medium innerhalb des Linsenkapselsackes, wobei das flüssige Medium an die zu entfernenden Epithelzellen angrenzt oder diese bedeckt, wobei die Druckpulse so gewählt oder beschaffen sind, dass die Epithelzellen durch die auftreffenden Druckpulse von der Wandung des Linsenkapselsackes abgelöst werden und zugleich in der Wandung des Linsenkapselsackes beim Entfernen der Epithelzellen keine Öffnung oder andere Beschädigungen, insbesondere durch die Druckpulse, entstehen. Bevorzugt wird ein Laserhandstück gemäß der US 5,324,282 A oder US 5,906,611 A verwendet, jedoch wird bei WO 2005/107665 A1 kein Unterdruck in der Hohlnadel erzeugt und kein Gewebe durch die Öffnung angesaugt. Vielmehr dient die Öffnung in der Hohlnadel bei WO 2005/107665 A1 nur zum Austritt der optisch erzeugten Druckpulse im Medium.

Es ist nun eine Aufgabe der Erfindung, einen Applikator und eine Vorrichtung zur Zellbehandlung, insbesondere zur Entfernung oder Inaktivierung von Epithelzellen, vorzugsweise am Linsenkapselsack eines Auges, insbesondere zur Zellbehandlung bei einem PCO, mittels durch Laserstrahlung an einem Target erzeugter Druckpulse anzugeben, mit der das Risiko einer Beeinträchtigung oder Beschädigung von umgebendem, nicht zu behandelndem Gewebe, insbesondere eine Beschädigung des Linsenkapselsackes selbst, verringert wird.

Diese Aufgabe wird gelöst durch den Gegenstand des Patentanspruchs 1. Vorteilhafte Ausgestaltungen ergeben sich aus den abhängigen Ansprüchen.

Der Applikator gemäß Patentanspruch 1 ist ausgebildet und bestimmt zur Zellbehandlung, insbesondere zur Epithelzellenentfernung oder -inaktivierung, vorzugsweise am menschlichen oder tierischen Auge, durch Druckpulse, insbesondere Stoßwellen, und umfasst
a) eine Hohlnadel mit einer Wandung, wobei die Wandung einen Hohlraum umschließt und an einem geschlossenen Ende geschlossen ausgebildet ist,
b) wobei am geschlossenen Ende an der Innenseite der Wandung ein Target (oder: Beschussmaterial, Beschussziel) angeordnet oder ausgebildet ist,
c) wobei im Hohlraum der Hohlnadel vom Target und/oder vom geschlossenen Ende beabstandet ein Laserstrahlungsemitter zum Abstrahlen von, vorzugsweise gepulster, Laserstrahlung angeordnet ist,
d) wobei eine Hauptabstrahlrichtung des Laserstrahlungsemitters in Richtung auf das Target gerichtet ist oder wobei, mit anderen Worten, der Laserstrahlungsemitter so angeordnet ist, dass die austretende Laserstrahlung durch einen zwischen dem Laserstrahlungsemitter und dem Target befindlichen Zwischenraum direkt auf das Target trifft,
e) wobei am Target bei oder durch Beaufschlagung oder Bestrahlung des Targets mit Laserstrahlung des Laserstrahlungsemitters wenigstens ein Druckpuls, insbesondere durch optischen Durchbruch, unter Ausbildung eines Plasmas erzeugbar ist oder erzeugt wird,
f) wobei die Wandung der Hohlnadel eine seitliche (oder: laterale) Austrittsöffnung zum Austritt des Druckpulses aus dem Hohlraum der Hohlnadel aufweist und zwischen der seitlichen Austrittsöffnung und dem Target oder dem geschlossenen Ende vollständig geschlossen ist,
g) wobei die Wandung (20) am geschlossenen Ende (3) der Hohlnadel (2) einen Verschluss (21) aufweist, an dem das Target (13) angeordnet oder ausgebildet ist, wobei der Verschluss (21) an der Außenseite und/oder der Innenwandung (24) konvex und kuppelförmig ausgebildet ist,
h) wobei die Wandung (20) der Hohlnadel (2) im Anschluss an den Verschluss (21) im Wesentlichen eine zylindrische Mantelwandung (23) um die Längsachse (A) der Hohlnadel (2) als Zylinderachse bildet,
i) wobei die seitliche Austrittsöffnung (8) in der Mantelwandung (23) gebildet ist und
j) wobei die seitliche Austrittsöffnung (8) von dem Verschluss (21) der Hohlnadel (2) und dem Target (13) um einen zur Längsachse (A) axialen Abstand (a) beabstandet ist, der größer als ein Außendurchmesser (D der Mantelwandung (23) der Hohlnadel (2) ist.

Die seitliche Austrittsöffnung kann beispielsweise von dem Target um einen Abstand beabstandet sein, der größer als die mittlere freie Weglänge des Plasmas im Hohlraum der Hohlnadel ist und/oder der so groß gewählt ist, dass im Wesentlichen kein Plasma aus dem Hohlraum der Hohlnadel durch die seitliche Austrittsöffnung austritt.

Die Vorrichtung gemäß Patentanspruch 9 umfasst wenigstens einen Applikator gemäß der Erfindung und eine Laserstrahlungsquelle.

Die Erfindung beruht auf der Überlegung, das nicht zu zerstörende oder zu behandelnde Gewebe, insbesondere die Wandung des Linsenkapselsackes, vor einer Beaufschlagung mit Plasma, das am Target als laserinduziertes Plasma entsteht, zu schützen.

In der gängigen Anwendung wie der Linsenextraktion ist dieses laserinduzierte Plasma nicht schädlich, da es bei ohnehin zu zerstörendem Gewebe zu dessen Zerstörung eher beiträgt. In der speziellen, gemäß der Erfindung bevorzugten Anwendung einer Behandlung oder Vermeidung (Prophylaxe) eines PCO, bei der die Epithelzellen vom Linsenkapselsack abgelöst, zumindest aber im Wachstum gehemmt, werden sollen, darf das unmittelbar dahinter angrenzende dünne und freitragende und damit empfindliche Gewebe des Linsenkapselsackes aber nicht angegriffen werden.

Gemäß der Erfindung wird laserinduzierte Plasma bereits an einem Austritt aus der für die Druckpulse vorgesehenen Austrittsöffnung der Hohlnadel gehindert. Dazu wird gemäß der Erfindung in einer ersten Maßnahme die Hohlnadel an dem Ende mit dem Target verschlossen, was insbesondere bedeutet, dass, im Unterschied zum Stand der Technik, im entsprechenden axialen Stirnende der Hohlnadel gerade keine Öffnung vorhanden ist. In einer zweiten Maßnahme wird nun statt einer Austrittsöffnung am Ende der Hohlnadel eine seitliche (oder: laterale) Austrittsöffnung in einem ausreichend großen Abstand von dem Ende der Hohlnadel und dem dort angeordneten Target ausgebildet und angeordnet, so dass das Plasma nicht mehr aus der seitlichen Austrittsöffnung austreten kann, anders als beim Stand der Technik, bei dem die Austrittsöffnung an der Spitze der Hohlnadel angeordnet ist und direkt an das Target angrenzt. Zwar wird durch diese Maßnahmen gemäß der Erfindung prinzipiell auch eine Abschwächung der Intensität der an dieser Austrittsöffnung austretenden Druckpulse in Kauf genommen, jedoch ist dies bei Anwendungen, bei denen die Intensität der Druckpulse ohnehin nicht zu hoch gewählt werden darf, wie beispielsweise bei einer Kapselreinigung, akzeptabel.

Unter einem Druckpuls wird in der vorliegenden Anmeldung eine zeitlich begrenzte Druckerhöhung, insbesondere eine Druckströmung oder Druckwelle oder Stoßwelle, verstanden, die sich innerhalb des flüssigen Mediums zu den Epithelzellen hin ausbreitet. Hierbei kann neben einem Energietransport und einem Impulstransport wie bei einer Welle zusätzlich auch ein Materietransport wie bei einer Strömung oder einem Druckstrahl erfolgen.

Eine besonders vorteilhafte Anwendung finden der Applikator und die Vorrichtung zum Entfernen (oder: Abtragen, Ablösen, Abtrennen, Absprengen) oder zumindest Inaktivieren, d.h. insbesondere dem Inhibieren (Hemmen des Wachstums), der Epithelzellen von bzw. an der Innenwand des Linsenkapselsackes eines Auges.

Der Applikator ist bevorzugt als manuell handhabbares, insbesondere medizinisches oder chirurgisches Werkzeug, ausgebildet, das zur Zellbehandlung eines vorgegebenen Gewebebereichs in den Körper eines Organismus eingebracht werden kann. Die insbesondere chirurgische, Hohlnadel kann mit ihrer Spitze, z. B. perkutan, in den Organismus, z.B. durch die Hornhaut in die Augenlinse, eingebracht werden. Die Zellbehandlung kann, je nach Eindringtiefe, an kutanen, subkutanen oder weiter innen liegenden Zellen durchgeführt werden.

In einer vorteilhaften Ausführungsform umfasst der Laserstrahlungsemitter eine Laserstrahlungsleitfaser (kurz: Laserfaser oder auch optische Faser), deren freies Ende eine Abstrahlfläche für die Laserstrahlung bildet und deren zweites Ende mit einer Laserstrahlungsquelle zum Einkoppeln von deren Laserstrahlung direkt oder indirekt gekoppelt oder koppelbar ist. Die Laserstrahlungsleitfaser verläuft insbesondere im Hohlraum der Hohlnadel, vorzugsweise entlang deren Längsachse. Das die Abstrahlfläche bildende freie Ende der Laserstrahlungsleitfaser ist bevorzugt an einem der seitlichen Austrittsöffnung gegenüberliegenden und/oder von der seitlichen Austrittsöffnung weiter als die zentrale Längsachse beabstandet liegenden Bereich der Wandung der Hohlnadel angeordnet ist.

Weiterhin ist vorzugsweise die seitliche Austrittsöffnung, entlang der Längsachse der Hohlnadel und/oder der Hauptabstrahlungsrichtung des Laserstrahlungsemitters oder der Ausbreitungsrichtung der Druckpulse gesehen, zwischen dem geschlossenen Ende der Hohlnadel und/oder dem Target einerseits und der Abstrahlfläche des Laserstrahlungsemitters, insbesondere dem freien Ende der Laserstrahlungsleitfaser, andererseits angeordnet. Ferner ist insbesondere die Austrittsöffnung bezüglich einer die Längsachse der Hohlnadel enthaltenden Symmetrieebene symmetrisch ausgebildet und/oder angeordnet.

Vorzugsweise ist die Hauptabstrahlrichtung der Laserstrahlung parallel zu der Längsachse der Hohlnadel.

Alternativ oder auch zusätzlich kann in der Hohlnadel selbst auch ein aktiver Laserstrahlungsemitter verwendet werden. Unter einem aktiven Laserstrahlungsemitter soll dabei eine Laserstrahlungsquelle verstanden werden, welche unmittelbar Laserstrahlung erzeugt, wie beispielsweise eine oder ein Array von Laserdioden.

Das Target ist dazu ausgebildet und eingerichtet, bei Beaufschlagung mit Laserstrahlung am Target einen optischen Durchbruch zu erzeugen, der unter Ausbildung von Plasma, insbesondere im Medium, eine Druckwelle bzw. Stoßwelle erzeugt.

Das Target kann dabei gepulst mit Laserstrahlung, d. h. mit einzelnen Laserstrahlungspulsen, beaufschlagt werden. Eine gepulste Beaufschlagung soll bedeuten, dass eine entsprechende Laserstrahlungsquelle im Pulsbetrieb betrieben wird. Zur Anwendung in der Epithelzellenbehandlung können beispielsweise Pulsraten von 1 bis 10 Pulsen pro s für Laserstrahlung in Wellenlängenbereiche(n) von beispielsweise 532 bis 1064 nm verwendet werden.

Bevorzugt ist zumindest das Target, jedenfalls ein vom Laserstrahlung beaufschlagter, bzw. zu beaufschlagender Abschnitt, aus Metall, insbesondere Titan oder einer Titanlegierung, hergestellt. Hierbei ist es möglich, und es kann von Vorteil sein, dass die gesamte Hohlnadel, insbesondere deren Wandungen, aus Titan hergestellt ist. Es kommen aber auch andere inerte Materialien für das Target bzw. die Wandung der Hohlnadel in Betracht, so z.B. Zirkonium(legierung) oder Instrumentenstahl, z.B. 316 Stahl.

Zumindest bei der bestimmungsgemäßen Verwendung des Applikators ist bevorzugt die Hohlnadel zumindest in einem zwischen dem Target und der seitlichen Austrittsöffnung befindlichen Zwischen- oder Innenraum mit einem flüssigen Medium, z.B. Irrigationsflüssigkeit und/oder Salzlösung wie BSS (Ringlerlösung), zum Übertragen der Druckpulse gefüllt, wobei vorzugsweise das Plasma sich im flüssigen Medium ausbildet. Vorzugsweise befindet sich die Hohlnadel insgesamt in dem flüssigen Medium und/oder das flüssige Medium grenzt an die zu behandelnden Zellen, insbesondere Epithelzellen, an oder bedeckt diese, so dass die Druckpulse durch das flüssige Medium zu den Zellen propagieren können oder propagieren.

Die Wandung der Hohlnadel weist am geschlossenen Ende der Hohlnadel einen Verschluss auf, an dem das Target angeordnet oder ausgebildet ist, wobei der Verschluss an der Außenseite und/oder der Innenwandung konvex und kuppelförmig ausgebildet ist. Der Verschluss ist konvex und kuppelförmig, vorzugsweise im Wesentlichen sphärisch oder teilweise konisch und teilweise sphärisch oder nur konisch, ausgebildet und/oder bevorzugt nach außen gewölbt oder gekrümmt, insbesondere in Richtung einer Längsachse der Hohlnadel, wodurch vorzugsweise eine einer Linse vergleichbare Wirkung oder eine fokussierende Wirkung auf den Druckpuls, insbesondere die Stoßwelle, in Ausbreitungsrichtung vom Target hin zur Austrittsöffnung erreicht wird. Ein Krümmungsradius des gekrümmten Verschlusses ist dann im Allgemeinen abhängig von der Dicke oder dem Durchmesser der Hohlnadel und wird in der Regel kleiner oder gleich dem Durchmesser der Hohlnadel gewählt.

Die Wandung der Hohlnadel im Anschluss an das geschlossene Ende, insbesondere den Verschluss, ist im Wesentlichen hohlzylindrisch oder als zylindrische Mantelwandung, die um die Längsachse der Hohlnadel als Zylinderachse gebildet ist, geformt, wobei die seitliche Austrittsöffnung in dieser Mantelwandung gebildet ist.

Nach einer weiteren besonders vorteilhaften Ausgestaltung wird das geschlossene Ende durch einen monolithischen Verschluss der Hohlnadel gebildet. Darunter soll insbesondere verstanden werden, dass der Verschluss mit dem anschließenden Bereich der Hohlnadel monolithisch, d. h. in einem Stück und/oder aus einem Material, ausgebildet sind. In diesem Fall ist der Verschluss kein separates, an der Hohlnadel lösbar befestigtes Element. Der Verschluss kann in diesem Fall einen der Wandung der Hohlnadel zuzurechnenden, insbesondere gekrümmten, Abschnitt bilden.

Lediglich der Vollständigkeit halber wird darauf hingewiesen, dass das erste Ende auch durch einen mit der Hohlnadel lösbar verbundenen Verschluss, z. B. Pfropf, verschlossen sein kann. Die vorhin erwähnte monolithische Lösung bietet jedoch den Vorteil, dass keine Verbindung und keine lösbaren Teile vorhanden sind, die sich infolge der erzeugten Stoßwellen lösen könnten. Letzteres ist insbesondere von Relevanz, da die Hohlnadel in der Regel in den Organismus eingeführt werden muss.

Eine Beabstandung der seitlichen Austrittsöffnung von dem geschlossenen Ende der Hohlnadel und dem Target ist so eingestellt, dass der Abstand größer als ein Außendurchmesser der Mantelwandung der Hohlnadel ist.

In einer weiteren besonderen Ausgestaltung ist die Öffnung in Axialrichtung der Hohlnadel vom Target beabstandet und bevorzugt unmittelbar angrenzend an die Abstrahlfläche angeordnet. Unmittelbar angrenzend soll insbesondere bedeuten, dass eine vom geschlossenen Ende der Hohlnadel abgewandte erste Kante der Öffnung etwa eine zur Axialrichtung senkrechte Ebene durch die Abstrahlfläche berührt. Die senkrechte Ebene durch die Abstrahlfläche kann auch als Abstrahlebene bezeichnet werden.

In einer vorteilhaften Ausführungsform ist die Austrittsöffnung im Wesentlichen kreisrund ausgebildet mit einem Durchmesser um eine Mittelachse die vorzugsweise senkrecht zur Längsachse gerichtet ist. Der Durchmesser ist kleiner als der Außendurchmesser, jedoch bevorzugt größer als der Innendurchmesser der Hohlnadel, insbesondere der Mantelwandung. Bevorzugt ist eine solche Austrittsöffnung durch spanabhebendes Bohren durch die Wandung, insbesondere Mantelwandung, von außen mittels eines Bohrers in im Wesentlichen senkrecht zur Längsachse gerichteter Bohrrichtung hergestellt.

In einer besonders vorteilhaften Ausführungsform ist die Austrittsöffnung als Langloch ausgebildet, das sich mit einer Längsrichtung oder der (größeren) Längsabmessung parallel zur Längsachse (A) erstreckt und das insbesondere eine ovale Form oder auch eine Stadionform mit zwei durch geradlinige parallel zur Längsachse verlaufende Randsegmente verbundenen halbkreisförmigen Randsegmenten aufweist. Eine Querabmessung der Austrittsöffnung, die kleiner ist als die Längsabmessung, ist nun bevorzugt kleiner als der Außendurchmesser, jedoch vorzugsweise größer als der Innendurchmesser der Hohlnadel, insbesondere der Mantelwandung.

Insbesondere kann sich zwischen dem ersten Ende, d. h. dem Target, und der zweiten Kante der Öffnung ein topfartiges Volumen für ein bei optischem Durchbruch entstehendes Plasma ergeben. Die mittlere freie Weglänge des Plasmas ist kleiner als die axiale Erstreckung des topfartigen Volumens, so dass ein Austritt des Plasmas aus dem Inneren der Hohlnadel vermieden werden kann.

Eine weitere Ausgestaltung des Applikators sieht vor, dass die Hohlnadel vom axial offenen Enden zum geschlossenen Ende hin im Durchmesser, vorzugsweise stufenartig, abnimmt. Vorzugsweise ist ein Übergang bzw. Übergangsbereich zwischen zwei Segmenten mit unterschiedlichen Durchmessern konisch, d. h. als Kegelstumpf, ausgebildet. Bei entsprechenden Durchmessern der beiden Segmente bildet der Kegelstumpf ein zum geschlossenen Enden hin sich konisch verjüngendes Zwischensegment.

Nach einer Ausgestaltung des Applikators ist die Abstrahlfläche, sofern zutreffend der Durchmesser der optischen Faser, insbesondere der gesamten optischen Faser, bevorzugt kleiner, vorzugsweise deutlich kleiner, als der kleinste Innendurchmesser der Hohlnadel. Für die optische Faser ergibt sich für diese Konfiguration ein Zwischenraum zwischen Innenwandung der Hohlnadel und optischer Faser. Dieser Zwischenraum kann beispielsweise als Kommunikationskanal verwendet werden.

Insbesondere mit den erwähnten Ausgestaltungen können die Erzeugung des Plasmas, und damit eine Erzeugung einer Stoßwelle, sowie die Ausbreitung der Stoßwelle in vorteilhafter Weise beeinflusst werden, so dass sich außerhalb der Hohlnadel, insbesondere durch Ausbreitung der Stoßwelle durch die Öffnung, eine vergleichsweise vorteilhafte, insbesondere verbesserte, lithotriptische Wirkung ergibt.

Nach Anspruch 9 ist eine Vorrichtung zur in vivo Zelllithotripsie vorgesehen. Die Vorrichtung umfasst einen Applikator gemäß zumindest einer der beschriebenen Ausgestaltungen bzw. Konfigurationen. Wegen Vorteilen und vorteilhaften Wirkungen wird insbesondere auf die obigen Ausführungen verwiesen.

Die Vorrichtung kann eine außerhalb der Hohlnadel befindliche Laserstrahlungsquelle umfassen, welche zur Einkopplung von Laserstrahlung mit der optischen Faser optisch gekoppelt werden kann oder optisch gekoppelt ist. Ferner kann die Vorrichtung eine elektronische Steuerung zum Betrieb, insbesondere zum gepulsten Betrieb, der Laserstrahlungsquelle umfassen.

Nachfolgend werden mit Bezug zu den anhängenden Zeichnungen Ausführungsbeispiele der Erfindung näher beschrieben. Es zeigen:
- FIG 1: eine perspektivische Ansicht eines Applikators zur Zellbehandlung mit einer runden seitlichen Austrittsöffnung;
- FIG 2: eine Draufsicht auf den Applikator gemäß FIG 1;
- FIG 3: einen Axialschnitt des Applikators gemäß Linie III-III der FIG 2;
- FIG 4: ein vergrößertes in FIG 3 mit IV bezeichnetes Detail des vorderen Bereichs des Applikators in einem Axialschnitt,
- FIG 5: eine perspektivische Ansicht eines Applikators zur Zellbehandlung mit einer länglichen seitlichen Austrittsöffnung;
- FIG 6: eine Draufsicht auf den vorderen Bereich des Applikators gemäß FIG 5 und
- FIG 7: den vorderen Bereich des Applikators gemäß FIG 5 und 6 in einem Axialschnitt.

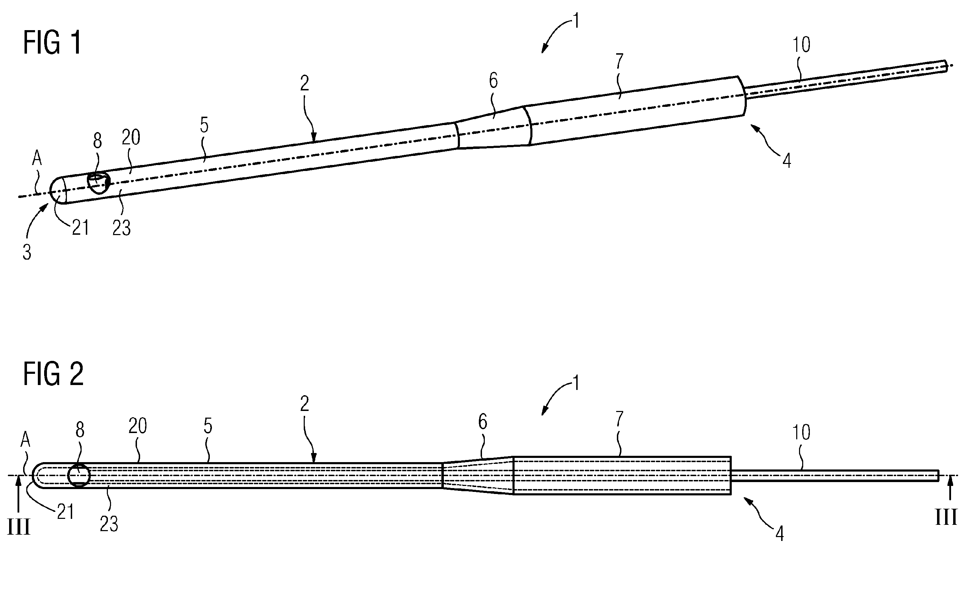

Einander entsprechende Teile und Größen sind in den FIG 1 bis 7 mit denselben Bezugszeichen versehen.

Die FIG 1 bis 7 zeigen Ausführungsbeispiele von Applikatoren 1 zur, im Allgemeinen *in vivo* stattfindenden, Zellbehandlung, insbesondere Zerstörung oder Inaktivierung von Epithelzellen am Linsenkapselsack eines Auges, z.B. für die Behandlung oder Vermeidung eines PCO. Der Applikator 1 ist grundsätzlich nicht auf die Behandlung von Epithelzellen beschränkt, jedoch dafür besonders geeignet.

Der Applikator 1 umfasst eine Hohlnadel 2, die an einem ersten, freien Ende 3 geschlossen ausgebildet ist und ein entgegen gesetztes zweites Ende 4 aufweist.

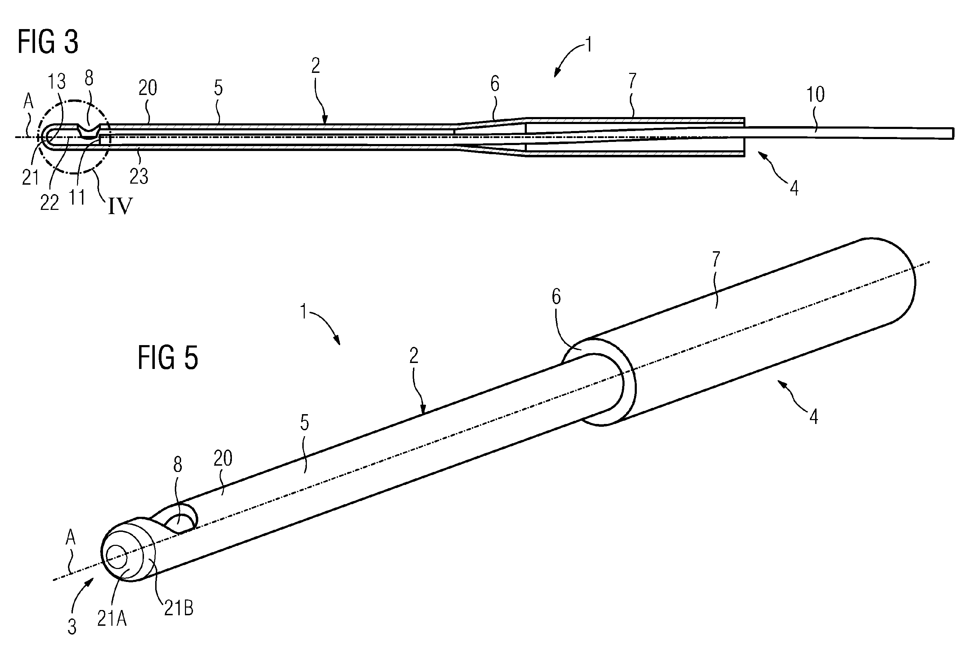

Die Hohlnadel 2 umfasst in Axialrichtung einer Längsachse A vom freien ersten Ende 3 ausgehend bis zu dem zweiten Ende 4, vorzugsweise in einstückiger Ausbildung, nacheinander geschaltet ein erstes Segment 5 mit dem ersten Ende 3, ein Zwischensegment 6 und ein zweites Segment 7 mit dem zweiten Ende 4. Das erste Segment 5 weist einen kleineren Außendurchmesser D auf als das zweite Segment 7, wodurch sich zum ersten Ende 3 hin eine im Außendurchmesser und vorzugsweise auch im Innendurchmesser abnehmende Konfiguration ergibt. Zur Überbrückung der unterschiedlichen Außendurchmesser des ersten Segments 5 und zweiten Segments 7 ist in FIG 1 bis 3 das Zwischensegment 6 zum ersten Ende 3 hin sich konisch verjüngend ausgebildet, während es in FIG 5 praktisch in Form einer Stufe oder stufenartig ausgebildet ist.

Die Wandung 20 der Hohlnadel 2 kann im ersten Segment 5 bei einer Wandstärke von etwa 0,1 mm einen Außendurchmesser D von etwa 0,8 mm, und im zweiten Segment 7 bei etwa gleicher Wandstärke einen Außendurchmesser von etwa 1,2 mm aufweisen. Bei den gegebenen Außendurchmessern kann das Zwischensegment 6, in Richtung vom ersten Ende 3 zum zweiten Ende 4 hin betrachtet, einen Öffnungswinkel von etwa 10 Grad aufweisen.

Die gesamte Hohlnadel 2 kann eine Länge von etwa 22,5 mm aufweisen, wobei das zweite Segment beispielsweise eine Länge von 7 mm aufweisen kann.

Die Wandung 20 der Hohlnadel 2 umschließt einen Hohlraum (oder: Kanal) 22 im Innern der Hohlnadel 2 und weist am freien ersten Ende 3 einen kuppelförmigen Verschluss 21 auf, an dem an der dem Hohlraum 22 zugewandten Innenseite ein Target (oder: Beschussmaterial) 13 angeordnet oder ausgebildet ist. Die Innenwandung des Verschlusses 21 ist mit 24 bezeichnet. Im Anschluss an den Verschluss 21 und das freie erste Ende 3 setzt sich die Wandung 20 der Hohlnadel 2 im ersten Segment 5 als zylindrische Mantelwandung 23 fort, die sich entlang der Längsachse A der Hohlnadel 2 (als Zylinderachse) erstreckt. Die Mantelwandung 23 weist einen axial gleichbleibenden Außendurchmesser D und einen axial gleichbleibenden Innendurchmesser y, der den Durchmesser des von der Mantelwandung 23 umschlossenen Bereichs des Hohlraumes 22 bildet, auf.

Das Target 13 ist vorzugsweise mit einem Teilbereich des Verschlusses 21 oder auch als Wandungsabschnitt der Wandung 20 gebildet und der Verschluss 21 besteht aus dem Target- oder Beschussmaterial des Targets 13. Ferner ist der Verschluss 21 vorzugsweise integral oder monolithisch mit den restlichen Bereichen oder aus dem gleichen Material wie die Mantelwandung 23 gebildet. Das Material des Targets 13 und ggf. des Verschlusses 21 sowie der Mantelwandung 23 ist vorzugsweise Titan oder eine Titanlegierung.

Während der *in vivo* Zellbehandlung befindet sich die Hohlnadel 2 im Allgemeinen in einem flüssigen Medium M, insbesondere einer Irrigationsflüssigkeit, und ist mit diesem gefüllt.

An der Innenseite der Mantelwandung 23 im Hohlraum 22 im ersten Segment 5 der Hohlnadel 2 liegt eine Laserstrahlungsleitfaser 10 (im Folgenden auch kurz: Faser 10) an, die parallel zur Längsachse A verläuft. Im Hohlraum 22 in dem Zwischensegment 6 und zweiten Segment 7 ist die Faser 10 nicht an der Wandung 20 der Hohlnadel 2 befestigt und tritt an dem zweiten Ende 4 der Hohlnadel 2, das offen ist, aus dem Hohlraum 22 der Hohlnadel 2 im Wesentlichen axial aus. Durch die lose Anordnung der Faser 10 in dem Zwischensegment 6 und dem zweiten Segment 7 ohne Befestigung an der Wandung 20 kann die Faser 10 frei gekrümmt oder gebogen verlaufen und dadurch flexibel ohne größere mechanische Spannungen an eine nicht dargestellte Kopplungsvorrichtung, insbesondere eine optische Steckverbindung, zum Koppeln mit einer nicht dargestellten Laserstrahlungsquelle zum Abstrahlen von Laserstrahlung L angekoppelt werden.

Der Applikator 1 bildet zusammen mit der Laserstrahlungsquelle und ggf. der Kopplungsvorrichtung eine Vorrichtung zur Zellbehandlung, insbesondere Epithelzellenbehandlung.

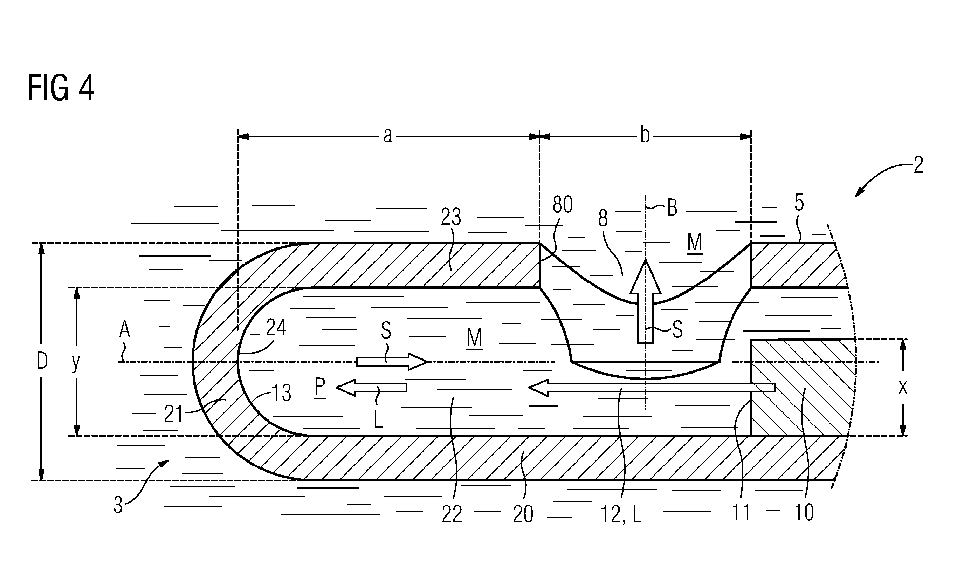

Die Laserstrahlungsleitfaser 10 überträgt die eingekoppelte Laserstrahlung L bis zu einem freien Ende der Faser, an dem die Laserstrahlung L aus der Faser 10 wieder austritt und dessen Stirnseite somit eine Abstrahlfläche 11 für die Laserstrahlung L bildet. Die Abstrahlfläche 11 liegt in FIG 4 in einer zur Längsachse A senkrechten Ebene. Eine Hauptabstrahlrichtung 12 der Laserstrahlung L ist in Richtung auf das freie erste Ende 3 auf das Target 13 am Verschluss 21 gerichtet. Die Hauptabstrahlrichtung 12 verläuft vorliegend parallel zur Längsachse A und somit orthogonal zur Abstrahlfläche 11. Folglich wird die aus der Faser 10 in den Hohlraum 22 austretende Laserstrahlung L durch den Zwischenraum im Hohlraum 22 zwischen Abstrahlfläche 11 und Target 13 in der Hauptabstrahlrichtung 12 zum Target 13 am Verschluss 21, im Allgemeinen durch das flüssige Medium M, das sich im Hohlraum 22 und auch außerhalb der Hohlnadel 2 befindet, übertragen und trifft auf das Targetmaterial.

Durch diese Beaufschlagung oder Bestrahlung des Targets 13 mit Laserstrahlung L, genauer mit Laserstrahlungspulsen, entsprechend hoher Energie, insbesondere 4 bis 12 mJ bei beispielsweise 1064 nm Wellenlänge, oder Leistung kann am Target 13 durch einen dabei entstehenden optischen Durchbruch oder Laserstrahlungsdurchbruch im Targetmaterial und ein damit einhergehend entstehendes Plasma P eine Stoßwelle bzw. Druckwelle S als Druckpuls, hier in dem flüssigen Medium M, erzeugt und weitergeleitet werden. Die Ausbreitungsrichtungen der Stoßwelle S sind mit zwei breiten Pfeilen veranschaulicht.

Die Hohlnadel 2 weist nun eine die Wandung 20 durchgreifende, vorzugsweise radial oder senkrecht zur Längsachse A nach außen zeigende, seitliche (oder: laterale) Austrittsöffnung 8 zum Austritt oder Durchtritt der Stoßwelle oder des Druckpulses S aus dem Hohlraum 22 der Hohlnadel 2 nach außen auf. Diese seitliche Austrittsöffnung 8 ist im Bereich des ersten Segments 5 in der Mantelwandung 23 der Hohlnadel 2 erzeugt oder angeordnet. Die Austrittsöfffnung 8 ist um eine, vorzugsweise senkrecht zur Längsachse A gerichtete radial zu dieser verlaufende, Mittelachse B ausgebildet.

Die Austrittsöffnung 8 ist oder liegt vorzugsweise bezüglich einer die Längsachse A der Hohlnadel 2 enthaltenden und vorzugsweise einer von der Längsachse A und der und die Mittelachse B aufgespannten Symmetrieebene symmetrisch.

Der von der Wandung 20 oder 23 gebildete Rand der Austrittsöffnung 8 ist mit 80 bezeichnet.

In FIG 1 bis 4 ist die Austrittsöffnung 8 in der Draufsicht oder im Querschnitt annähernd kreisrund mit einem Durchmesser (oder allgemein: einer lichten Weite) b ausgebildet, der kleiner als der Außendurchmesser D, jedoch im Allgemeinen größer als der Innendurchmesser y der Hohlnadel 2 oder der Mantelwandung 23 ist, also y < b < D.

Eine solche runde Austrittsöffnung 8 kann durch spanabhebendes Bohren durch die Mantelwandung 23 von außen mittels eines Bohrers in im Wesentlichen senkrecht zur Längsachse A gerichteter Bohrrichtung hergestellt werden.

Der Rand 80 der Austrittsöffnung 8 ergibt sich aus dem geometrischen Schnitt aus einem Zylinder des Durchmessers b entlang der Mittelachse B durch den Zylinder des Durchmessers D entlang der Längsachse A der Mantelwandung 23 und ist deshalb haubenförmig ausgebildet wie dargestellt.

In FIG 5 bis 7 ist die laterale Austrittsöffnung 8 dagegen als Langloch ausgebildet, das sich mit einer Längsrichtung parallel zur Längsachse A erstreckt. Die Längsabmessung der Austrittsöffnung 8 parallel zur Längsachse A ist mit c und die Querabmessung der Austrittsöffnung 8 senkrecht zur Längsachse A und zur Längsabmessung c ist mit d bezeichnet. Der Rand 80 der Austrittsöffnung 8 ist in dieser Ausführungsform in zwei durch geradlinige parallel zur Längsachse A verlaufende Randsegmente 80A verbundene halbkreisförmige Randsegmente 80B gebildet, so dass sich eine stadionartige Form ergibt.

Hingewiesen sei darauf, dass die Abmessungen wie die lichte Weite b oder die Längsabmessung c und die Querabmessung d der Austrittsöffnung 8 in Abhängigkeit der erzeugten oder zu erzeugenden Stoßwellen verändert bzw. angepasst sind.

Der Verschluss 21 ist konvex, insbesondere kuppelförmig, ausgebildet. Gemäß FIG 1 bis 4 ist der Verschluss 21, vorzugsweise im Wesentlichen sphärisch, insbesondere mit einem Mittelpunkt auf der Längsachse A, nach außen gewölbt oder gekrümmt, insbesondere in Richtung der Längsachse A. Es bildet also die Außenwandung des Verschlusses 21 eine kugelförmige stumpfe Spitze der Hohlnadel 2. Auch die Innenwandung 24 des Verschlusses 21 mit dem Target 13 ist, wie in FIG 4 zu erkennen, sphärisch ausgebildet, vorzugsweise mit einem Mittelpunkt auf der Längsachse A, vorzugsweise demselben Mittelpunkt wie die Außenwandung.

Gemäß FIG 5 bis 7 ist der Verschluss 21 an seiner Außenseite aus einem konisch um die Längsachse A geformten äußeren Teilwandungsbereich (oder: Außenkonus) 21A mit dem Außenkonuswinkel α, der zwischen 80° und 100°, insbesondere bei etwa 90°, gewählt ist, und einem zentralen sphärisch gekrümmten inneren Teilwandungsbereich 21B, der eine stumpfe Spitze der Hohlnadel 2 bildet, zusammengesetzt. Die Teilwandungsbereiche 21 A ud 21B gehen glatt (stetig differenzierbar) ineinander über. An der Innenseite oder Innenwandung 24 des Verschlusses 21 ist eine Innenkonusform mit einer Spitze und einem Innenkonuswinkel β, der zwischen 100° und 160°, insbesondere bei etwa 120°, gewählt ist, verwirklicht, die insbesondere mit einer Bohrerspitze beim Bohren des Hohlraumes 22 erzeugt werden kann.

Bei beiden Formen des Verschlusses 21 wird infolge der Krümmung oder zumindest der Konvexität eine einer Linse vergleichbare fokussierende Wirkung auf den Druckpuls, insbesondere die Stoßwelle S, in Ausbreitungsrichtung vom Target 13 hin zur seitlichen Austrittsöffnung 8 erreicht. Es entsteht auch in beiden Fällen ein stumpfes freies Ende 3 der Hohlnadel 2, was die Gefahr einer Verletzung von Gewebe beim Bewegen der Hohlnadel 2 reduziert.

Der Druckpuls oder die Stoßwelle S pflanzt sich vom Target 13 zunächst durch den Hohlraum 22 und dann durch die seitliche Austrittsöffnung 8 hinweg nach außen in das auch außerhalb der Hohlnadel 2 befindliche Medium M fort und kann außerhalb der Hohlnadel 2 bei einem zur Austrittsöffnung 8 benachbarten Gewebe zur Zellbehandlung, insbesondere Zerstörung, Inaktivierung und/oder Entfernung entsprechender Zellen, insbesondere Epithelzellen am Linsenkapselsack des Auges, verwendet werden.

Die seitliche Austrittsöffnung 8 ist nun mit ihrem Rand 80 in Axialrichtung zur Längsachse A vom ersten Ende 3 bzw. der Innenseite des Verschlusses 21 oder dem Target 13 um einen axialen Abstand a beabstandet bzw. ihre Mittelachse B ist um einen Abstand a + b/2 beabstandet.

Vorzugsweise ist der Abstand a zwischen dem ersten Ende 3 und der Öffnung 8 größer als die mittlere freie Weglänge des Plasmas P gewählt, das am Target 13 entsteht. Auf diese Weise kann in vorteilhafter Weise ein Austreten des Plasmas P aus der Austrittsöffnung 8, zumindest weitgehend, vermieden werden, was sich positiv auf die zellbehandelnde Wirkung des Applikators 1 auswirken kann, da das Plasma P nicht das umliegende nicht zu behandelnde Zellgewebe, aber auch nicht das zu behandelnde Zellgewebe direkt angreifen kann. Somit wird das zu behandelnde Zellgewebe nur durch die Stoßwelle S im umgebenden Medium M beaufschlagt.

Der Abstand a ist größer als der Innendurchmesser y des Hohlraumes 22 der Hohlnadel 2, also a > y, und vorzugsweise auch größer als der Außendurchmesser D der Hohlnadel 2, also a > D, gewählt sowie ferner auch größer als der Durchmesser oder die Längsabmessung b der Austrittsöffnung 8 gewählt, also a > b. Absolute Werte für den Abstand a liegen bevorzugt bei wenigstens 0,7 mm, vorzugsweise zwischen 0,8 mm und 1,9 mm. Ferner ist der Abstand a im Allgemeinen größer gewählt, wenn die Energie des Laserstrahlungspulses höher ist und/oder der Puls kürzer ist, und kleiner, wenn die Energie kleiner bzw. der Puls länger ist.

Wie insbesondere aus FIG 4 ersichtlich ist, ist die Außenabmessung der Abstrahlfläche 11, und vorliegend auch der Durchmesser x der Faser 10, kleiner als der Innendurchmesser y des ersten Segments 5. Aufgrund der vorliegenden Geometrien ist der Durchmesser x der Faser 10 auch kleiner als die Innendurchmesser des Zwischensegments 6 und des zweiten Segments 7. Ein geeigneter Faserdurchmesser x ist etwa 350 µm.

Wie in FIG 4 gezeigt, ist die Abstrahlfläche 11, und damit einhergehend zumindest ein anschließender Abschnitt der Faser 10, auf der der Austrittsöffnung 8 gegenüberliegenden Innenseite der Wandung 20 und deren Mantelwandung 23 gelegen und/oder in einem axialen Abstand von dem Target 13 oder der Innenseite des Verschlusses 21 angeordnet, welcher axiale Abstand mindestens a + b beträgt, vorzugsweise gleich a + b ist, also bündig mit dem vom Target abgewandten Rand der Austrittsöffnung 8 abschließt. Es wird dadurch vermieden, dass Laserstrahlung L direkt aus der Öffnung 8 austreten kann.

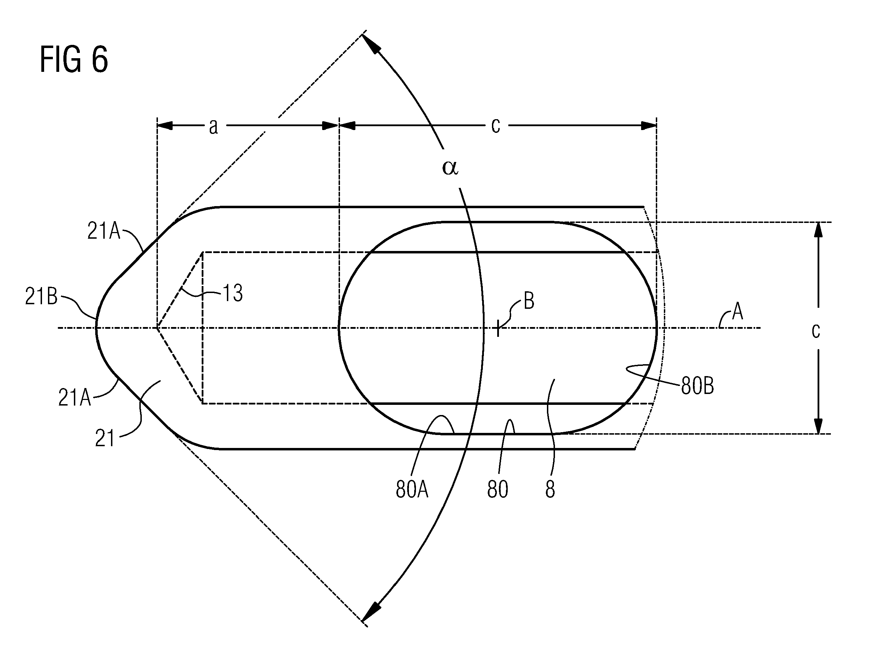

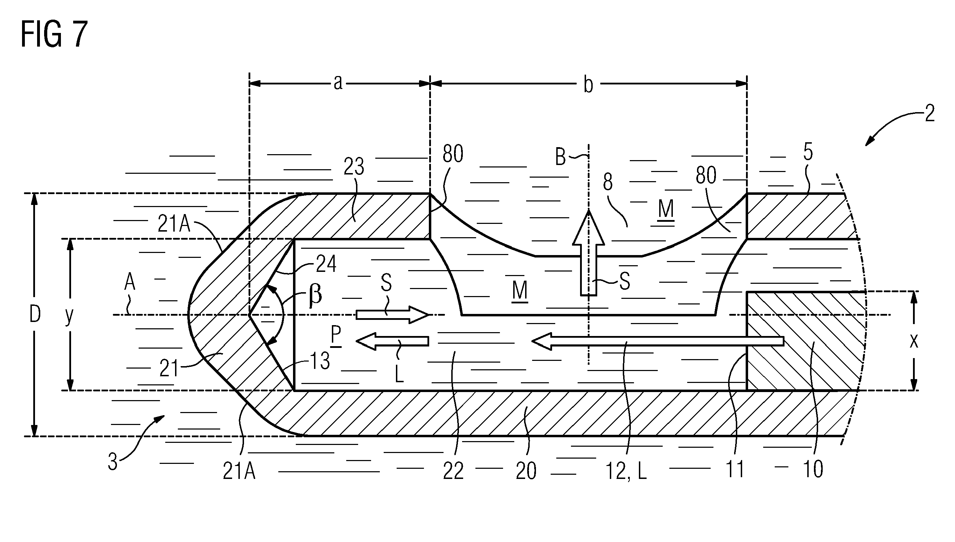

In FIG 6 und 7 ist entsprechend der axiale Abstand der Abstrahlfläche 11 von dem Target 13 oder der Innenwandung 24 gleich oder größer a + c. Insbesondere mit den angegebenen geometrischen Verhältnissen ergibt sich zwischen dem ersten Ende 3 und der Austrittsöffnung 8 ein in der Form etwa topf- oder becherförmiger Bereich innerhalb der Hohlnadel 2. Dieser becherförmige Bereich ist insoweit von Vorteil, als das Plasma zumindest teilweise darin eingeschlossen bleibt, d. h. ein Austreten aus der Hohlnadel im Wesentlichen verhindert wird. Bei entsprechender Strahlungsleistung der Laserstrahlungsquelle wird ein Plasma erzeugt, dessen freie Weglänge kleiner ist als die axiale Länge des becherförmigen Bereichs, und es wird erreicht, dass das Plasma im wesentlichen Umfang in der Hohlnadel 2 verbleibt.

Um Beschädigungen der Faser 10 zu vermeiden, sind die Kanten der Hohlnadel 2, d. h. des zweiten Segments 7, im Bereich des zweiten Endes 4, gratfrei ausgebildet.

Da optische Fasern vergleichsweise wenig Bauraum benötigen, können im Querschnitt vergleichsweise dünne Hohlnadeln für den Applikator verwendet werden. Es soll jedoch erwähnt werden, dass auch die Verwendung von Laserstrahlungsquellen innerhalb der Hohlnadel möglich ist, sofern ausreichend Bauraum vorhanden ist.

Im Folgenden wird als besonders vorteilhafte Anwendung des Applikators und der Vorrichtung eine Katarakt-Operation mit Post-Katarakt-Prophylaxe beschrieben. Eine Katarakt-Operation umfasst in der Regel die folgenden Verfahrensschritte:

Zunächst wird mittels eines chirurgischen Instruments, beispielsweise einer Kanüle, der vordere Kapselsack eröffnet, wobei eine in der Regel 4,5 mm bis 5,5 mm große Öffnung erzeugt wird *(Capsulorrhexis).* Sodann werden in der Regel an entgegen gesetzten Seiten Inzisionen in der Hornhaut *(cornea)*, insbesondere am *Limbus*, für ein photolytisches Laserhandstück einerseits und ein Irrigationshandstück andererseits erzeugt. Durch Einbringen einer Spülflüssigkeit, z.B. BSS, wird die Augenlinse vom Kapselsack gelöst und dadurch mobilisiert (Hydrodisektion). Mittels des Irrigationsinstruments wird nun Spülflüssigkeit, im Allgemeinen ebenfalls BSS, in den Kapselsack eingespült und durch den dadurch aufgebauten Druck der Spülflüssigkeit insbesondere verhindert, dass die hintere Wandung des Kapselsackes zu nahe an das Laserhandstück gelangt, und zugleich der Kapselsack gespült.

Vorzugsweise mittels des Applikators gemäß der Erfindung oder ggf. auch eines anderen Applikators, der beispielsweise ein Laserhandstück der Firma A.R.C. Laser GmbH sein kann oder gemäß der eingangs genannten US 5,324,282 A oder US 5,906,611 A aufgebaut sein kann, wird die Augenlinse sukzessive durch die mittels Laserpulse erzeugten Druckpulse oder Schockwellen photolytisch abgebaut und abgesaugt. Dabei kann die Augenlinse, insbesondere deren zum Schluss bearbeiteter Nukleus, mittels des Irrigationswerkzeugs bewegt und in seiner Position relativ zum Applikator optimiert werden. Mittels einer Vielzahl von Laserpulsen und der dadurch ausgelösten Schockwellen wird das Gewebe der Linse Stück für Stück zerstört und die einzelnen Gewebeteile können abgesaugt werden. Im Anschluss wird nach vollständiger Entfernung der natürlichen Augenlinse nun eine künstliche Augenlinse in den Kapselsack eingesetzt. Der Chirurg arbeitet mit beiden Instrumenten in bimanueller Technik.

Gemäß der Erfindung wird nun entweder vor Einsetzen der künstlichen Linse oder vorzugsweise nach Einsetzen der künstlichen Linse, da dann die künstliche Linse die durch die *Capsulorrhexis* erzeugte Öffnung im vorderen Kapselsack verschließt, mittels des Applikators oder einer Vorrichtung gemäß der Erfindung die Kapselsackinnenseite von Epithelzellen gereinigt zur Vermeidung eines PCO.

Nun ist die Wandung des Linsenkapselsackes relativ dünn und empfindlich. Dadurch ist der Linsenkapselsack nach Entfernen der natürlichen Augenlinse nicht in der Lage, seine Form zu halten, so dass selbst bei Einbringen von Spülflüssigkeit dennoch der Kapselsack wenigstens teilweise in sich zusammenfallen kann und dadurch die Gefahr einer Beschädigung durch den Applikator besteht.

Es hat sich zwar schon gezeigt, dass die erzeugten, vergleichsweise starken Schockwellen oder Druckpulse für die Kapselsackwandung in der Regel dennoch unschädlich sind, wenn die Kapselsackwandung hinreichend entspannt ist und durch Verformung nachgeben kann. Es ist also zu vermeiden, dass die Kapselsackwandung unter Spannung steht oder gewissermaßen steif ist, wenn die Schockwelle zur Entfernung oder Absprengung der Epithelzellen auftrifft. Deshalb wird während der Epithelzellenentfernung keine oder nur eine geringe Absaugfunktion verwendet und kein oder nur ein geringer Unterdruck mittels einer Pumpe oder dergleichen erzeugt, damit nicht die Schockwelle auf die Kapselsackwandung im stark angesaugten Zustand treffen würde oder bereits aufgrund des Unterdrucks beim Absaugen die Kapselsackwandung eingesaugt würde und ein Riss oder eine Öffnung entstünde.

Im Allgemeinen wird auch die Pulswiederholungsrate für die Laserstrahlungspulse, d.h. die Zahl der pro Zeiteinheit, beispielsweise Sekunde, applizierten Laserstrahlungspulse so gering gehalten, dass die Kapselsackwandung sich nach einem Laserstrahlungspuls und einer dadurch erzeugten Schockwelle wieder vor dem nächsten Laserstrahlungspuls und der zugehörigen Schockwelle in einen entspannten Zustand zurückbewegen oder zurückschwingen kann und sich nicht beim Auftreffen des nächsten Laserstrahlungspulses gerade in der verformten und damit unter (maximaler) Spannung stehenden Lage befindet, in der der Kapselsack anfällig ist zu reißen. Der Kapselsack sollte also sozusagen mit den repetierten Druckpulsen mitschwingen können.

Gemäß der Erfindung wurde überraschenderweise erkannt, dass auch das Plasma, das bei der optische Erzeugung der Druckpulse mittels der Laserstrahlung entsteht, und bei bekannten Applikatoren aus dem Applikator austritt, durch das flüssige Medium auf die Kapselsackwandung treffen kann und dort überraschenderweise zu zellularen Schäden auch an dem den Epithelzellen benachbarten Gewebe führen kann.

Gemäß der Erfindung wird deshalb durch die beschriebene besondere konstruktive Ausbildung der Hohlnadel des Applikators ein solcher Austritt von Plasma im Wesentlichen verhindert. Zugleich bleibt aber die Stärke der Druckpulse ausreichend, um die Epithelzellen abzutragen.

Somit ist in dieser bevorzugte Anwendung eine schonende und wirkungsvolle Entfernung oder zumindest Inaktivierung von Epithelzellen am Linsenkaspelsack eines Auges, insbesondere im Rahmen einer Prophylaxe eines Sekundärkatarakts oder PCO, mit Hilfe des Applikators und der zugehörigen Vorrichtung erreicht.

Die Pulswiederholungsrate der Druckpulse der Vorrichtung wird, vorzugsweise bereits an der Bedieneinheit, durch eine entsprechende Ansteuerung der Laserstrahlungspulse insbesondere begrenzt auf maximal 10 Hz, das heißt 10 Pulse pro Sekunde, insbesondere höchstens 4 Hz. Es sind grundsätzlich aber auch höhere Frequenzen möglich. Nach unten ist keine Beschränkung erforderlich und es können Pulswiederholungsraten bis nahezu 0 Hz eingestellt werden. Die Druckpulse können Stoßwellen oder Druckwellen sein oder auch mit Materialtransport verbundene Druckströmungen oder Druckstrahlen sein.

Das Frequenzspektrum der Druckwelle vor der Ausbildung der Stoßwelle durch nicht lineare Effekte kann vom Bereich einiger Hz bis in den Bereich von 100 kHz gehen, wodurch neben Schallschwingungen im hörbaren Bereich auch Ultraschallwellen oder -schwingungen möglich sind.

### Bezugszeichenliste

- 1: Applikator
- 2: Hohlnadel
- 3: erstes Ende
- 4: zweites Ende
- 5: erstes Segment
- 6: Zwischensegment
- 7: zweites Segment
- 8: Austrittsöffnung
- 10: Faser
- 11: Abstrahlfläche
- 12: Hauptabstrahlrichtung
- 13: Target
- 20: Wandung
- 21: Verschluss
- 21A: Teilwandung
- 21B: Teilwandung
- 22: Hohlraum
- 23: Mantelwandung
- 24: Innenwandung
- 75: Übergang
- 80: Rand
- 80A: Randsegment
- 80B: Randsegment

- A: Längsachse
- B: Mittelachse
- a: Abstand
- b: Durchmesser
- c: Längsabmessung
- d: Querabmessung
- D: Außendurchmesser
- x: Faserdurchmesser
- y: Innendurchmesser
- L: Laserstrahlung
- M: flüssiges Medium
- P: Plasma
- S: Druckpuls, insbesondere Stoßwelle
- α: Außenkonuswinkel
- β: Innenkonuswinkel

## Patentansprüche

1. Applikator (1) ausgebildet und bestimmt zur Zellbehandlung, insbesondere zur Epithelzellenentfernung oder -inaktivierung, vorzugsweise am menschlichen oder tierischen Auge, durch Druckpulse und
a) umfassend eine Hohlnadel (2) mit einer Wandung (20), die einen Hohlraum (22) umschließt und an einem geschlossenen Ende (3) geschlossen ausgebildet ist,
b) wobei am geschlossenen Ende (3) an der Innenseite der Wandung (20) ein Target (13) angeordnet oder ausgebildet ist,
c) wobei im Hohlraum (20) der Hohlnadel (2) vom Target (13) und/oder vom geschlossenen Ende (3) beabstandet ein Laserstrahlungsemitter (10, 11) zum Abstrahlen von, vorzugsweise gepulster, Laserstrahlung (L) angeordnet ist,
d) wobei der Laserstrahlungsemitter (10, 11) so angeordnet ist, dass die austretende Laserstrahlung (L) durch einen zwischen dem Laserstrahlungsemitter (10, 11) und dem Target (13) befindlichen Zwischenraum hindurch direkt auf das Target (13) trifft,
e) wobei durch Beaufschlagung des Targets (13) mit Laserstrahlung (L) des Laserstrahlungsemitters (10, 11) am Target (13) unter Ausbildung eines Plasmas (P) wenigstens ein Druckpuls (S) erzeugbar ist oder erzeugt wird,
f) wobei die Wandung (20) der Hohlnadel (2) eine seitliche Austrittsöffnung (8) zum Austritt des Druckpulses (S) aus dem Hohlraum (22) der Hohlnadel (2) aufweist und zwischen der seitlichen Austrittsöffnung (8) und dem Target (13) oder dem geschlossenen Ende (3) vollständig geschlossen ist,
g) wobei die Wandung (20) am geschlossenen Ende (3) der Hohlnadel (2) einen Verschluss (21) aufweist, an dem das Target (13) angeordnet oder ausgebildet ist, wobei der Verschluss (21) an der Außenseite und/oder der Innenwandung (24) konvex und kuppelförmig ausgebildet ist,
h) wobei die Wandung (20) der Hohlnadel (2) im Anschluss an den Verschluss (21) im Wesentlichen eine zylindrische Mantelwandung (23) um die Längsachse (A) der Hohlnadel (2) als Zylinderachse bildet,
i) wobei die seitliche Austrittsöffnung (8) in der Mantelwandung (23) gebildet ist und
j) wobei die seitliche Austrittsöffnung (8) von dem Verschluss (21) der Hohlnadel (2) und dem Target (13) um einen zur Längsachse (A) axialen Abstand (a) beabstandet ist, der größer als ein Außendurchmesser (D der Mantelwandung (23) der Hohlnadel (2) ist.

2. Applikator nach Anspruch 1, bei dem die Hohlnadel (2) zumindest in einem zwischen dem Target (13) und der seitlichen Austrittsöffnung (8) befindlichen Zwischenraum mit einem flüssigen Medium zum Übertragen der Druckpulse (S) gefüllt ist, wobei vorzugsweise das Plasma (P) sich im flüssigen Medium (M) ausbildet.

3. Applikator nach Anspruch 1 oder Anspruch 2, bei dem der Verschluss (21) zumindest teilweise im Wesentlichen sphärisch, gewölbt oder gekrümmt ist, und/oder wenigstens konisch geformt ist, insbesondere in Richtung einer Längsachse (A) der Hohlnadel (2).

4. Applikator nach einem der vorhergehenden Ansprüche, bei dem der Laserstrahlungsemitter eine Laserstrahlungsleitfaser (10) umfasst, deren freies Ende eine Abstrahlfläche (11) für die Laserstrahlung (L) bildet und deren zweites Ende mit einer Laserstrahlungsquelle gekoppelt oder koppelbar ist zum Einkoppeln von Laserstrahlung der Laserstrahlungsquelle in die Laserstrahlungsleitfaser (10), wobei die Laserstrahlungsleitfaser (10) insbesondere im Hohlraum (22) der Hohlnadel (2), vorzugsweise entlang deren Längsachse (A), verläuft und/oder wobei das die Abstrahlfläche (11) bildende freie Ende der Laserstrahlungsleitfaser (10) an einem der seitlichen Austrittsöffnung (8) gegenüberliegenden und/oder von der seitlichen Austrittsöffnung (8) weiter als die zentrale Längsachse (A) beabstandet liegenden Bereich der Wandung (20) der Hohlnadel (2) angeordnet ist und/oder wobei vorzugsweise die seitliche Austrittsöffnung (8), entlang der Längsachse (A) der Hohlnadel (2) gesehen, zwischen dem geschlossenen Ende (3), insbesondere Verschluss (21), der Hohlnadel (2) und/oder dem Target (13) einerseits und der Abstrahlfläche (11) des Laserstrahlungsemitters (10, 11), insbesondere dem freien Ende der Laserstrahlungsleitfaser (10), andererseits angeordnet ist.

5. Applikator nach einem der vorhergehenden Ansprüche, bei dem die Austrittsöffnung (8) bezüglich einer die Längsachse (9) der Hohlnadel (2) und vorzugsweise auch eine Mittelachse (B) der Austrittsöffnung (8) enthaltenden Symmetrieebene symmetrisch ist oder liegt.

6. Applikator nach einem der Ansprüche 1 bis 5, bei dem die Austrittsöffnung (8) im Wesentlichen kreisrund mit einem Durchmesser (b) um eine Mittelachse (B), die vorzugsweise senkrecht zur Längsachse (A) gerichtet ist, ausgebildet ist, wobei im Allgemeinen der Durchmesser (b) kleiner als der Außendurchmesser (D), jedoch bevorzugt größer als der Innendurchmesser (y) der Hohlnadel (2), insbesondere der Mantelwandung (23), ist und/oder wobei bevorzugt die Austrittsöffnung (8) durch spanabhebendes Bohren durch die Wandung (20) insbesondere Mantelwandung (23), von außen mittels eines Bohrers in im Wesentlichen senkrecht zur Längsachse (A) gerichteter Bohrrichtung hergestellt ist.

7. Applikator nach einem der Ansprüche 1 bis 6, bei dem die Austrittsöffnung (8) als Langloch ausgebildet ist, das sich mit einer Längsrichtung oder der Längsabmessung (c) parallel zur Längsachse A erstreckt und das insbesondere eine ovale Form oder auch eine Stadionform mit zwei durch geradlinige parallel zur Längsachse (A) verlaufende Randsegmente (80A) verbundenen halbkreisförmigen Randsegmenten (80B) aufweist,
wobei im Allgemeinen eine Querabmessung (d) der Austrittsöffnung (8), die kleiner ist als die Längsabmessung (c) kleiner als der Außendurchmesser (D), jedoch bevorzugt größer als der Innendurchmesser (y) der Hohlnadel (2), insbesondere der Mantelwandung (23), ist.

8. Applikator nach einem der vorhergehenden Ansprüche, bei dem die Hohlnadel (2) vom zweiten Ende (4) zum geschlossenen ersten Ende (3) hin im Durchmesser abnimmt, wobei ein Übergang zwischen zwei Segmenten (5, 7) jeweils konstanten, aber zueinander unterschiedlichen Durchmessers durch ein zum geschlossenen Ende hin sich konisch verjüngendes oder auch eine Stufe bildendes Zwischensegment (6) ausgebildet ist, wobei vorzugsweise die Lichtstrahlungsleitfaser (10) im ersten Segment (5) an der Wandung (20) befestigt ist und im zweiten Segment (7) und Zwischensegment (6) nicht an der Wandung (20) befestigt ist, und/oder wobei ein das geschlossene Ende (3) umfassendes axiales erstes Segment (5) der Hohlnadel (2) einen kleineren Außendurchmesser, ein das offene Ende (4) umfassendes axiales zweites Segment (7) der Hohlnadel (2) einen größeren Außendurchmesser, aufweisen.

9. Vorrichtung zur Anwendung oder ausgebildet und bestimmt zur Zellbehandlung, insbesondere zur Epithelzellenentfernung oder -inaktivierung, vorzugsweise am menschlichen oder tierischen Auge, durch Druckpulse, insbesondere Stoßwellen (S), umfassend einen Applikator (1) nach einem der Ansprüche 1 bis 10 und wenigstens eine Laserstrahlungsquelle zum Erzeugen von Laserstrahlung (L).

10. Vorrichtung nach Anspruch 9, wobei die Laserstrahlung gepulst ist mit einer Pulsdauer zwischen 5 ns und 20 ns, vorzugsweise von 8 ns bis 12 ns, und/oder einer Pulsenergie zwischen 1 und 20 mJ, vorzugsweise zwischen 4 und 12 mJ, wobei jeder Laserpuls wenigstens einen Druckpuls erzeugt, und/oder wobei die Hohlnadel (2) zumindest überwiegend mit dem flüssigen Medium gefüllt ist und in diesem angeordnet ist.

## Claims

1. Applicator (1) formed and intended for cell treatment, in particular for epithelial cell removal or inactivation, preferably in the human or animal eye, by pressure pulses and
a) comprising a hollow needle (2) with a wall (20) which encloses a cavity (22) and is formed to be closed at a closed end (3),
b) wherein a target (13) is arranged or formed at the closed end (3) on the inner side of the wall (20),
c) wherein a laser radiation emitter (10, 11) for radiating preferably pulsed laser radiation (L) is arranged in the cavity (20) of the hollow needle (2) spaced apart from the target (13) and/or from the closed end (3),
d) wherein the laser radiation emitter (10, 11) is arranged so that the emergent laser radiation (L) directly strikes the target (13) through an intermediate space located between the laser radiation emitter (10, 11) and the target (13),
e) wherein at least one pressure pulse (S) can be generated or is generated by exposing the target (13) to laser radiation (L) of the laser radiation emitter (10, 11) on the target (13) with formation of a plasma (P),
f) wherein the wall (20) of the hollow needle (2) has a lateral outlet opening (8) for outlet of the pressure pulse (S) out of the cavity (22) of the hollow needle (2) and is entirely closed between the lateral outlet opening (8) and the target (13) or the closed end (3),
g) wherein the wall (20) has, at the closed end (3) of the hollow needle (2), a closure (21) on which the target (13) is arranged or formed, wherein the closure (21) is formed to be convex and dome-shaped on the outside and/or the inner wall (24),
h) wherein the wall (20) of the hollow needle (2), following on from the closure (21), forms substantially a cylindrical liner wall (23) around the longitudinal axis (A) of the hollow needle (2) as a cylinder axis,
i) wherein the lateral outlet opening (8) is formed in the liner wall (23) and
j) wherein the lateral outlet opening (8) is spaced apart from the closure (21) of the hollow needle (2) and the target (13) by an axial distance (a) to the longitudinal axis (A) which is larger than an outer diameter (D) of the liner wall (23) of the hollow needle (2).

2. Applicator according to Claim 1, in which the hollow needle (2) is filled with a liquid medium for transmission of the pressure pulses (S) at least in an intermediate space located between the target (13) and the lateral outlet opening (8), wherein the plasma (P) is preferably formed in the liquid medium (M).

3. Applicator according to Claim 1 or Claim 2, in which the closure (21) is at least partially substantially spherical, arched or curved, and/or is shaped at least conically, in particular in the direction of a longitudinal axis (A) of the hollow needle (2).

4. Applicator according to any one of the preceding claims, in which the laser radiation emitter comprises a laser radiation conducting fibre (10), the free end of which forms a radiating surface (11) for the laser radiation (L) and the second end of which is coupled or can be coupled to a laser radiation source for coupling laser radiation of the laser radiation source into the laser radiation conducting fibre (10), wherein the laser radiation conducting fibre (10) runs in particular in the cavity (22) of the hollow needle (2), preferably along its longitudinal axis (A), and/or wherein the free end of the laser radiation conducting fibre (10) which forms the radiating surface (11) is arranged on a region of the wall (20) of the hollow needle (2) situated opposite the lateral outlet opening (8) and/or spaced apart further than the central longitudinal axis (A) from the lateral outlet opening (8) and/or wherein preferably the lateral outlet opening (8), as seen along the longitudinal axis (A) of the hollow needle (2), is arranged between the closed end (3), in particular closure (21), of the hollow needle (2) and/or the target (13) on one hand and the radiating surface (11) of the laser radiation emitter (10, 11), in particular the free end of the laser radiation conducting fibre (10) on the other hand.

5. Applicator according to any one of the preceding claims, in which the outlet opening (8) is or lies symmetrical in relation to a plane of symmetry which contains the longitudinal axis (9) of the hollow needle (2) and preferably also a central axis (B) of the outlet opening (8).

6. Applicator according to any one of Claims 1 to 5, in which the outlet opening (8) is formed to be substantially circular with a diameter (b) around a central axis (B), which is preferably directed perpendicular to the longitudinal axis (A), wherein the diameter (b) is generally smaller than the outer diameter (D), but preferably larger than the inner diameter (y) of the hollow needle (2), in particular of the liner wall (23), and/or wherein the outlet opening (8) is preferably produced by machining boring through the wall (20), in particular liner wall (23), from the outside by means of a borer in a boring direction directed substantially perpendicular to the longitudinal axis (A).

7. Applicator according to any one of Claims 1 to 6, in which the outlet opening (8) is formed as an elongated hole which extends parallel to longitudinal axis A with a longitudinal direction or the length dimension (c) and which has in particular an oval shape or also a stadium shape with two semi-circular edge segments (80B) connected by straight-lined edge segments (80A) running parallel to the longitudinal axis (A), wherein in general a transverse dimension (d) of the outlet opening (8), which is smaller than the length dimension (c) smaller than the outer diameter (D), but preferably larger than the inner diameter (y) of the hollow needle (2), in particular of the liner wall (23).

8. Applicator according to any one of the preceding claims, in which the diameter of the hollow needle (2) reduces from the second end (4) towards the closed first end (3), wherein a transition between two segments (5, 7) with in each case a constant, but different diameter from one another is formed by an intermediate segment (6) which tapers conically towards the closed end or also forms a step, wherein preferably the light radiation conducting fibre (10) is fastened in the first segment (5) to the wall (20) and in the second segment (7) and intermediate segment (6) is not fastened to the wall (20), and/or wherein an axial first segment (5) of the hollow needle (2) surrounding the closed end (3) has a smaller outer diameter, while an axial second segment (7) of the hollow needle (2) surrounding the open end (4) has a larger outer diameter.

9. Device for use or formed and intended for cell treatment, in particular for epithelial cell removal or inactivation, preferably in the human or animal eye, by pressure pulses, in particular shock waves (S), comprising an applicator (1) according to any one of Claims 1 to 10 and at least one laser radiation source for generation of laser radiation (L).

10. Device according to Claim 9, wherein the laser radiation is pulsed with a pulse duration between 5 ns and 20 ns, preferably from 8 ns to 12 ns, and/or a pulse energy between 1 and 20 mJ, preferably between 4 and 12 mJ, wherein each laser pulse generates at least one pressure pulse and/or wherein the hollow needle (2) is filled at least primarily with the liquid medium and is arranged therein.

## Revendications

1. Applicateur (1) conçu et destiné pour le traitement de cellules, plus particulièrement pour l'élimination ou l'inactivation de cellules épithéliales, de préférence au niveau de l'oeil humain ou animal, par des impulsions de pression et
a) comprenant une aiguille creuse (2) avec une paroi (20) qui entoure un espace creux (22) et qui est fermée au niveau d'une extrémité fermée (3),
b) une cible (13) étant disposée ou formée au niveau de l'extrémité fermée (3) à l'intérieur de la paroi (20),
c) dans l'espace creux (20) de l'aiguille creuse (2), un émetteur de rayon laser (10, 11) étant disposé à une certaine distance de la cible (13) et/ou de l'extrémité fermée (3) pour l'émission d'un rayon laser (L), de préférence pulsé,
d) l'émetteur de rayon laser (10, 11) étant disposé de façon à ce que le rayon laser (L) sortant arrive directement sur la cible (13) à travers un espace intermédiaire se trouvant entre l'émetteur de rayon laser (10, 11) et la cible,
e) grâce à l'irradiation de la cible (13) avec un rayon laser (L) de l'émetteur de rayon laser (10, 11) au niveau de la cible (13) avec la formation d'un plasma (P), au moins une impulsion de pression (S) pouvant être générée ou étant générée,
f) la paroi (20) de l'aiguille creuse (2) comprenant une ouverture de sortie latérale (8) pour la sortie de l'impulsion de pression (S) hors de l'espace creux (22) de l'aiguille creuse (2) et étant fermée complètement entre l'ouverture de sortie latérale (8) et la cible (13) ou l'extrémité fermée (3),
g) la paroi (20) comprenant, au niveau de l'extrémité (3) de l'aiguille creuse (2), une fermeture (21) au niveau de laquelle la cible (13) est disposée ou formée, la fermeture (21) étant formée de manière convexe et sous la forme d'une coupole à l'extérieur et/ou au niveau de la paroi interne (24),
h) la paroi (20) de l'aiguille creuse (2) formant globalement, après la fermeture (21), une enveloppe cylindrique (23) autour de l'axe longitudinal (A) de l'aiguille creuse (2) en tant qu'axe de cylindre,
i) l'ouverture de sortie latérale (8) étant formée dans l'enveloppe (23) et
j) l'ouverture de sortie latérale (8) étant située, par rapport à la fermeture (21) de l'aiguille creuse (2) et de la cible (13), à une distance axiale (a) par rapport à l'axe longitudinal (A) qui est supérieure à un diamètre extérieur (D) de l'enveloppe (23) de l'aiguille creuse (2).

2. Applicateur selon la revendication 1, dans lequel l'aiguille creuse (2) est remplie, au moins dans un espace creux se trouvant entre la cible (13) et l'ouverture de sortie latérale (8), avec un milieu liquide pour la transmission des impulsions de pression (S), le plasma (P) se formant de préférence dans le milieu liquide (M).

3. Applicateur selon la revendication 1 ou la revendication 2, dans lequel la fermeture (21) est au moins partiellement globalement sphérique, bombée ou incurvée et/ou au moins de forme conique, plus particulièrement en direction d'un axe longitudinal (A) de l'aiguille creuse (2).

4. Applicateur selon l'une des revendications précédentes, dans lequel l'émetteur de rayon laser comprend une fibre de guidage de rayon laser (10) dont une extrémité libre forme une surface d'émission (11) pour le rayon laser (L) et dont une deuxième extrémité est couplée ou peut être couplée avec une source de rayon laser pour l'introduction d'un rayon laser de la source de rayon laser dans la fibre de guidage de rayon laser (10), la fibre de guidage de rayon laser (10) s'étendant dans l'espace creux (22) de l'aiguille creuse (2), plus particulièrement le long de son axe longitudinal (A) et/ou l'extrémité libre de la fibre de guidage de rayon laser (10) formant la surface d'émission (11) étant disposée au niveau d'une zone située en face de l'ouverture de sortie latérale (8) et/ou au niveau d'une zone située à une distance de l'ouverture de sortie latérale (8) supérieure à l'axe longitudinal central (A) de la paroi (20) de l'aiguille creuse (2) et/ou, de préférence, l'ouverture de sortie latérale (8) étant disposée, vue le long de l'axe longitudinal (A) de l'aiguille creuse (2), entre l'extrémité fermée (3), plus particulièrement la fermeture (21) de l'aiguille creuse (2) et/ou la cible (13), d'une part, et la surface d'émission (11) de l'émetteur de rayon laser (10, 11), plus particulièrement l'extrémité libre de la fibre de guidage de rayon laser (10), d'autre part.

5. Applicateur selon l'une des revendications précédentes, dans lequel l'ouverture de sortie (8) est symétrique par rapport à un plan de symétrie contenant l'axe longitudinal (9) de l'aiguille creuse (2) et de préférence également un axe central (B) de l'ouverture de sortie (8).

6. Applicateur selon l'une des revendications 1 à 5, dans lequel l'ouverture de sortie (8) est de forme globalement circulaire avec un diamètre (b) autour d'un axe central (B), qui est de préférence perpendiculaire à l'axe longitudinal (A), le diamètre (b) étant généralement inférieur au diamètre extérieur (D), mais de préférence supérieur au diamètre intérieur (y) de l'aiguille creuse (2), plus particulièrement de l'enveloppe (23) et/ou, de préférence, l'ouverture de sortie (8) étant réalisée par alésage à enlèvement de copeaux à travers la paroi (20), plus particulièrement l'enveloppe (23), de l'extérieur au moyen d'une perceuse dans une direction de perçage globalement perpendiculaire à l'axe longitudinal (A).

7. Applicateur selon l'une des revendications 1 à 6, dans lequel l'ouverture de sortie (8) est conçue comme un trou oblong qui s'étend, avec une direction longitudinale ou la dimension longitudinale (c), parallèlement à l'axe longitudinal (A) et qui présente plus particulièrement une forme ovale ou une forme de stade avec deux segments de bords (80B) de forme semi-circulaire reliés entre aux par des segments de bords (80A) linéaires s'étendant parallèlement à l'axe longitudinal (A), une dimension transversale (d) de l'ouverture de sortie (8) étant généralement inférieure à la dimension longitudinale (c), inférieure au diamètre extérieur (D), mais, de préférence, supérieure au diamètre intérieur (y) de l'aiguille creuse (2), plus particulièrement de l'enveloppe (23).

8. Applicateur selon l'une des revendications précédentes, dans lequel le diamètre de l'aiguille creuse (2) diminue de la deuxième extrémité (4) vers la première extrémité fermée (3), une transition entre deux segments (5, 7) de diamètres constants mais différents entre eux étant formée par un segment intermédiaire (6) conique se rétrécissant en direction de l'extrémité fermée ou formant un épaulement, la fibre de guidage de rayon laser (10) étant fixée de préférence dans le premier segment (5) sur la paroi (20) et n'étant pas fixée sur la paroi (20) dans la deuxième segment (7) et le segment intermédiaire (6) et/ou un premier segment (5) axial comprenant l'extrémité fermée (3), présentant un diamètre extérieur inférieur et un deuxième segment (7) axial de l'aiguille creuse (2), comprenant l'extrémité ouverte (4), présentant un diamètre extérieur supérieur.

9. Dispositif pour l'application ou conçu et destiné au traitement de cellules, plus particulièrement pour l'élimination ou l'inactivation de cellules épithéliales, de préférence dans l'oeil humain ou animal, par des impulsions de pression, plus particulièrement des ondes de choc (S), comprenant un applicateur (1) selon l'une des revendications 1 à 10 et au moins une source de rayon laser pour la production d'un rayon laser (L).

10. Dispositif selon la revendication 9, le rayon laser étant pulsé avec une durée d'impulsion entre 5 ns et 20 ns, de préférence de 8 ns à 12 ns et/ou une énergie d'impulsion entre 1 et 20 mJ, de préférence entre 4 et 12 mJ, chaque impulsion laser produisant au moins une impulsion de pression et/ou l'aiguille creuse (2) étant remplie au moins majoritairement d'un milieu liquide ou étant disposée dans celui-ci.
